# EUROPEAN PATENT SPECIFICATION

(11) **EP 2 956 144 B1**
(45) Date of publication and mention of the grant of the patent: **22.11.2017**
(21) Application number: 14751940.9
(22) Date of filing: 14.02.2014
(51) Int. Cl.: A61K 9/00, A61K 9/107, A61K 31/573, A61K 33/30, A61P 27/14, A61P 37/08

(54) **DIFLUPREDNATE EMULSION COMPOSITION CONTAINING ZINC**
DIFLUPREDNATEMULSIONSZUSAMMENSETZUNG MIT ZINK
COMPOSITION D'ÉMULSION À BASE DE DIFLUPRÉDNATE CONTENANT DU ZINC

(30) Priority: 15.02.2013 US 201361765307 P
(43) Date of publication of application: 23.12.2015
(73) Proprietor: Senju Pharmaceutical Co., Ltd., Osaka-shi, Osaka 541-0046 (JP)
(72) Inventor: IEMOTO, Suzuka, Kobe-shi Hyogo 651-2241 (JP); YAMAGUCHI, Masazumi, Kobe-shi Hyogo 651-2241 (JP); YASUEDA, Shinichi, Kobe-shi Hyogo 651-2241 (JP)
(74) Representative: Vossius & Partner Patentanwälte Rechtsanwälte mbB
(86) International application number: PCT/JP2014/054097
(87) International publication number: WO 2014/126266

(56) References cited:
- WO-A1-98/30221
- JP-A- H1 129 483
- JP-A- 2003 104 870
- JP-A- 2007 530 685
- JP-A- 2010 504 358
- JP-A- 2010 504 990
- US-A- 6 114 319
- US-A1- 2001 003 003
- US-A1- 2007 212 420
- US-A1- 2007 212 420
- US-A1- 2010 021 562
- Anonymous: "Durezol (difluprednate ophthalmic emulsion) 0.05%", , June 2012 (2012-06), pages 4-11, XP055279148, internet Retrieved from the Internet: URL:http://www.accessdata.fda.gov/drugsatf da_docs/label/2012/022212s003lbl.pdf [retrieved on 2016-06-09]

## Description

### Technical Field

The present invention relates to a difluprednate emulsion composition containing zinc. More particularly, the present invention relates to a difluprednate-containing emulsion composition which is stable and has excellent preservative efficacy since it contains zinc.

### Background of the Invention

Difluprednate is a strong steroidal anti-inflammatory drug, and US Patent No. 6114319 describes an emulsion composition comprising difluprednate, which can be used as eye drop, nasal drop or ear drop. An emulsion composition comprising difluprednate shows excellent permeability of the drug into a lesion, and is advantageous in that it can be used as an eye drop causing extremely less uncomfortable feeling and extremely less foreign sensation upon administration.

Since eye drop, nasal drop and ear drop are preparations for repeated use except single-use formulations, even if they are sterilized before uncapping, the risk of contamination of the uncapped drug solution with microorganisms such as bacteria and the like is extremely high. To prevent such secondary contamination, therefore, benzalkonium chloride, chlorhexidine gluconate and the like are generally added as preservatives for aqueous liquids. These preservatives generally used for aqueous liquids cannot provide satisfactory preservative efficacy for emulsions, and therefore, preservatives with higher concentrations are necessary. However, since preservatives have been suggested to cause corneal problems and the like, the use of a high concentration preservative is not generally desirable.

Moreover, an application for drug approval requires submission of various data that guarantee quality, and one of them is a stability test. As the stability test, long-term test, accelerated test and photostability test are performed according to the guideline for stability test and the like (ICH: International Conference on Harmonisation of Technical Requirements for Registration of Pharmaceuticals for Human Use, Q1A and Q1B). These data are important for setting storage conditions and shelf life of drug products.

On the other hand, WO2008/036847 and WO2005/097067 describe aqueous ophthalmic compositions containing zinc ion.

### Summary of the Invention

The present invention aims to provide a stable emulsion composition comprising difluprednate, which has excellent preservative efficacy.

The present inventors have found that a stable emulsion composition comprising difluprednate, which has excellent preservative efficacy, can be prepared by adding zinc.

Accordingly, the present invention provides the following.
[1] An emulsion composition comprising difluprednate and zinc.
[2] The emulsion composition of the aforementioned [1], wherein zinc is a zinc salt or a zinc complex.
[3] The emulsion composition of the aforementioned [2], wherein the zinc salt or zinc complex is at least one selected from the group consisting of zinc sulfate and zinc chloride.
[4] The emulsion composition of the aforementioned [2] or [3], wherein the zinc salt or zinc complex has a concentration as zinc ion of not less than 0.00001 (w/v)% and not more than 0.1 (w/v)%.
[5] The emulsion composition of the aforementioned [3], further comprising sorbic acid and boric acid.
[6] The emulsion composition of any one of the aforementioned [1] to [5], which is an ophthalmic composition.
[7] A method of stabilizing an emulsion composition comprising difluprednate, the method comprising preparing an emulsion composition comprising difluprednate and zinc.
[8] The method of the aforementioned [7], wherein zinc is a zinc salt or a zinc complex.
[9] The method of the aforementioned [8], wherein the zinc salt or zinc complex is at least one selected from the group consisting of zinc sulfate and zinc chloride.
[10] The method of the aforementioned [8] or [9], wherein the zinc salt or zinc complex is added to the emulsion composition at a concentration of not less than 0.00001 (w/v)% and not more than 0.1 (w/v)% as zinc ion.
[11] The method of the aforementioned [9], further comprising adding sorbic acid and boric acid to the emulsion composition.
[12] The method of any one of the aforementioned [7] to [11], wherein the emulsion composition is an ophthalmic composition.

According to the present invention, a difluprednate-containing emulsion composition having excellent preservative efficacy and excellent stability (photostability and heat stability) can be provided.

In addition, a decrease in the viable cell count in 24 hours, namely, eradication of invaded microorganisms in a short time, is considered important in the preservative efficacy test in the European Pharmacopoeia. According to the present invention, an emulsion composition comprising difluprednate, which shows immediate effectiveness of the preservative efficacy, can be provided by adding sorbic acid and boric acid in addition to zinc.

### Detailed Description of the Invention

The present invention provides an emulsion composition comprising difluprednate and zinc. More particularly, the present invention provides an oil-in-water emulsion composition comprising difluprednate, oil, water, an emulsifier, and zinc (hereinafter to be referred to as the composition of the present invention). The composition of the present invention may further contain sorbic acid and boric acid.

Difluprednate (6α,9α-difluoroprednisolone 17-butyrate 21-acetate), which can be used for the composition of the present invention, is a steroidal anti-inflammatory drug known to show an excellent anti-inflammatory action and an excellent antiallergic action by transdermal administration or ocular instillation administration. Difluprednate can be prepared, for example, based on the methods described in US Patent No. 3780177 and US Patent No. 3784692.

Oils that can be used for the composition of the present invention may be any as long as they are low toxic, low irritative and applicable to the eye. Preferable examples include those containing fatty acid esters of glycerol, such as castor oil, peanut oil, cottonseed oil, soybean oil, olive oil, medium-chain triglyceride [e.g., Miglyol (trade name, Mitsuba Trading Co., Ltd.)] and the like. More preferred are, for example, castor oil, medium-chain triglyceride (e.g., Miglyol) and the like, which can dissolve difluprednate well, and particularly preferred is castor oil.

Water that can be used for the composition of the present invention is not particularly limited as long as it is generally added to pharmaceutical compositions, and purified water, distilled water for injection and the like can be mentioned.

As the kind of emulsifier that can be used for the composition of the present invention, non-ionic surfactant and the like can be mentioned. Examples thereof include polyoxyethylene sorbitan ester of fatty acids, polyoxyethylene hydrogenated castor oils, alkyl aryl polyether alcohol type polymers, polyoxyethylene fatty acid esters, polyoxyethylene polyoxypropylene glycols and sucrose fatty acid esters. Preferred are polyoxyethylene sorbitan monooleate, polyoxyethylene sorbitan monolaurate, polyoxyethylene sorbitan monopalmitate, polyoxyethylene sorbitan monostearate, polyoxyethylene sorbitan tristearate, polyoxyethylene hydrogenated castor oil 10, polyoxyethylene hydrogenated castor oil 40, polyoxyethylene hydrogenated castor oil 50, polyoxyethylene hydrogenated castor oil 60, tyloxapol, polyoxyl stearate and the like, and particularly preferred are polysorbate 80, polyoxyethylene hydrogenated castor oil 60, tyloxapol and polyoxyl 40 stearate. These may be used in combination.

The zinc in the composition of the present invention is preferably a compound that can be zinc ion in an emulsion composition. It may be in the form of a zinc salt or a zinc complex.

Examples of the compound that can be zinc ion include metal zinc, zinc salt, zinc complex and the like.

The metal zinc may be nanoparticulate zinc or colloidal zinc.

Examples of the zinc salt include zinc sulfate, zinc chloride, zinc oxide, zinc acetate, zinc carbonate, zinc hydroxide, zinc citrate, zinc lactate, zinc phosphate, zinc gluconate and the like. The zinc salt may be a hydrate thereof.

Examples of the zinc complex include zinc-ethanolamine, zinc-dimethyldithiocarbamate, zinc-sulfate, zinc-2-ethylhexanoate, zinc-quaternary alkylammonium, zinc-nitrate, zinc-8-quinolinolate, zinc amino acid complex and the like.

A preferable example of the zinc salt or zinc complex includes at least one selected from the group consisting of zinc sulfate and zinc chloride.

A preferable example of the composition of the present invention includes a composition free of an antimicrobial metal other than zinc.

The amount of difluprednate to be contained in the composition of the present invention is not less than 0.001 (w/v)%, preferably not less than 0.005 (w/v)%, more preferably not less than 0.01 (w/v)%, and not more than 0.4 (w/v)%, preferably not more than 0.3 (w/v)%, more preferably not more than 0.2 (w/v)%, of the composition. The most preferable amount of difluprednate to be added is 0.05 (w/v)%.

The amount of oil to be contained in the composition of the present invention is not particularly limited as long as it can generally provide an oil-in-water emulsion. The amount of oil in the composition is not less than 0.1 (w/v)%, preferably not less than 0.5 (w/v)%, more preferably not less than 1 (w/v)%, and not more than 30 (w/v)%, preferably not more than 20 (w/v)%, more preferably not more than 10 (w/v)%.

The amount of water to be contained in the composition of the present invention is not particularly limited, it is not less than 20 (w/v)%, preferably not less than 50 (w/v)%, more preferably not less than 60 (w/v)%, and not more than 99.8 (w/v)%, preferably not more than 99 (w/v)%, more preferably not more than 98 (w/v)%, of the composition.

The amount of an emulsifier to be contained in the composition of the present invention is not particularly limited as long as it can generally provide an oil-in-water emulsion. The amount of an emulsifier in the composition is not less than 0.1 (w/v)%, preferably not less than 0.5 (w/v)%, more preferably not less than 1 (w/v)%, and not more than 40 (w/v)%, preferably not more than 30 (w/v)%, more preferably not more than 20 (w/v)%.

The amount of zinc to be contained in the composition of the present invention, when it is a zinc salt or a zinc complex, is generally not less than about 0.00001 (w/v)% and not more than about 0.1 (w/v)%, preferably not less than about 0.0005 (w/v) and not more than about 0.1 (w/v), more preferably not less than about 0.001 (w/v)% and not more than about 0.1 (w/v)%, as a concentration of zinc ion in the composition.

While the amount of sorbic acid to be contained in the composition of the present invention is not particularly limited, it is not less than 0.01 (w/v)% and not more than 0.5 (w/v)%, preferably not less than 0.05 (w/v)% and not more than 0.2 (w/v)%, more preferably 0.1 (w/v)%, in the composition.

While the amount of boric acid to be contained in the composition of the present invention is not particularly limited, it is not less than 0.05 (w/v)% and not more than 0.5 (w/v)%, preferably not less than 0.1 (w/v)% and not more than 0.5 (w/v)%, more preferably 0.1 (w/v)%, in the composition.

While the combination of the amounts of the above-mentioned components in the composition of the present invention is not particularly limited, a combination of, for example, 0.001 - 0.4 (w/v)% of difluprednate, 0.1 - 40 (w/v)% of oil, 20 - 99.8 (w/v)% of water, 0.1 - 40 (w/v)% of an emulsifier, and a zinc salt or zinc complex at a concentration as zinc ion of about 0.00001 - about 0.1 (w/v)%, in the composition, preferably difluprednate 0.005 - 0.3 (w/v)%, oil 0.5 - 30 (w/v)%, water 50 - 99 (w/v)%, emulsifier 0.5 - 30 (w/v)%, and a zinc salt or zinc complex at a concentration as zinc ion of about 0.0005 - about 0.1 (w/v)%, in the composition, particularly preferably difluprednate 0.01 - 0.2 (w/v)%, oil 1 - 20 (w/v)%, water 60 - 98 (w/v)%, emulsifier 1 - 20 (w/v)%, and a zinc salt or zinc complex at a concentration as zinc ion of about 0.001 - about 0.1 (w/v)%, in the composition. Most preferably, the composition of the present invention contains the respective components at difluprednate 0.05 (w/v)%, castor oil 5.0 (w/v)%, polysorbate 80 4.0 (w/v)%, and a zinc salt or zinc complex at a concentration as zinc ion of about 0.001 - about 0.1 (w/v)%, and has pH about 7. Each of the above-mentioned compositions may further contain sorbic acid 0.1 (w/v)% and boric acid 0.1 (w/v)%.

The composition of the present invention can contain a water-soluble polymer to increase the stability of emulsion particles. Examples of the water-soluble polymer include povidone (polyvinylpyrrolidone), polyvinyl alcohol, hydroxyethylcellulose, hydroxypropylcellulose, methylcellulose, hydroxypropylmethylcellulose, carboxymethylcellulose, carboxyvinyl polymer, and salts thereof and the like. The water-soluble polymer can be added in about 0.001 - about 3 (w/v)% to the composition.

The composition of the present invention can contain a tonicity agent. Examples of the tonicity agent include boric acid, sodium chloride, potassium chloride, concentrated glycerol, propylene glycol, D-mannitol and the like. The above-mentioned isotonic agents can be added as long as they do not remarkably decrease the storage stability of difluprednate, and do not impair the physical stability of the emulsion. Particularly, the tonicity agent is preferably any of boric acid, sodium chloride and concentrated glycerol, which do not easily influence the storage stability of difluprednate. These may be used in combination.

The composition of the present invention is adjusted to have an osmotic pressure of about 150 - about 1100 mOsm, preferably about 150 - about 650 mOsm, more preferably about 220 - about 480 mOsm, by the addition of a tonicity agent as mentioned above.

The composition of the present invention can contain a buffering agent. Examples of the buffering agent include acetate salts such as sodium acetate and the like, phosphate salts such as monosodium dihydrogen phosphate, disodium monohydrogen phosphate, monopotassium dihydrogen phosphate, dipotassium monohydrogen phosphate and the like, amino acid salts such as ε-aminocaproic acid, sodium glutamate and the like, citric acid and a salt thereof, tromethamol, 4-(2-hydroxyethyl)-1-piperazineethanesulfonic acid and the like.

The buffering agent can be added as long as it does not decrease the storage stability of difluprednate, and the physical stability of the emulsion is not impaired. The buffering agent can be added in about 0.01 - about 2 (w/v)% of the composition.

It is also possible to add anti-inflammatory agents such as antiphlogistic agent, non-steroidal anti-inflammatory agent, anti-inflammatory analgesic agent and anti-inflammatory enzyme preparation, antiviral agent, antibacterial agent, antifungal agent, antiallergic agent, antibiotic, sulfa drug, synthetic penicillin, therapeutic agent for glaucoma, therapeutic agent for cataract, miotic agent, mydriatic agent, topical astringent, vasoconstrictor, agent to prevent intraocular pressure elevation, therapeutic agent for ocular hypertension, surface anesthetic, α1-blocker, β-blocked, β1-blocker, carbonate dehydratase inhibitor, topical selective H1-blocker, adrenal cortex hormone, vitamin B12, coenzyme type vitamin B2, anticholinesterase, organic iodine preparation and the like to the composition of the present invention.

The composition of the present invention can additionally contain various additives such as stabilizer, antioxidant, chelating agent, pH adjuster, thickener and the like. In addition, a preservative other than zinc can be further added. Examples of the antioxidant include ascorbic acid and a salt thereof, tocopherol, sodium thiosulfate, sodium hydrogen sulfite, pyruvic acid and a salt thereof and the like. Examples of the chelating agent include sodium edetate, citric acid and a salt thereof and the like. Examples of the pH adjuster include hydrochloric acid, phosphoric acid, acetic acid, sulfuric acid, boric acid, borax, sodium hydroxide, potassium hydroxide, sodium carbonate, sodium hydrogen carbonate, aqueous ammonia and the like. Particularly, examples of the acidic pH adjuster include hydrochloric acid, phosphoric acid, acetic acid, sulfuric acid and boric acid. Examples of the stabilizer include dibutylhydroxytoluene, tromethamol, sodium formaldehyde sulfoxylate, tocopherol, sodium pyrosulfite, monoethanolamine, aluminum monostearate, glycerol monostearate and the like. Examples of the thickener include carboxyvinyl polymer, hydroxyethylcellulose, hydroxypropylmethylcellulose, methylcellulose, alginic acid, polyvinyl alcohol, polyvinylpyrrolidone, macrogol, sodium hyaluronate and the like. Examples of the preservative that can be added other than zinc include benzalkonium chloride, benzethonium chloride, chlorhexidine gluconate, chlorobutanol, sorbic acid, potassium sorbate, sodium dehydroacetate, methyl p-hydroxybenzoate, ethyl p-hydroxybenzoate, propyl p-hydroxybenzoate, butyl p-hydroxybenzoate and the like.

The composition of the present invention can be provided as an aqueous preparation such as an oil-in-water (O/W) emulsion, a microemulsion and the like.

The average particle size (median size) of an oil drop of the composition of the present invention is preferably 10 - 2000 nm, more preferably 20 - 1000 nm, particularly preferably 20 - 500 nm. The average particle size can be measured by a particle size distribution measuring apparatus.

The composition of the present invention preferably has pH 4 - 8, more preferably pH 5 - 8, still more preferably pH 6.0 - 8.0, most preferably pH about 7.

In the present specification, unless otherwise specified, having immediate effectiveness of the preservative efficacy means decreasing the viable cell counts of bacteria and fungi in a short time in, for example, a preservative efficacy test. For example, it means that the viable cell count of bacteria (*Staphylococcus aureus, Escherichia coli* and *Pseudomonas aeruginosa*) decreases by 1 log or more 24 hr after inoculation, decreases by 3 log or more 7 days after inoculation, and does not increase 28 days after inoculation from the level of 7 days after inoculation, and the viable cell count of fungi *(Candida albicans* and *Aspergillus brasiliensis (niger))* decreases by 1 log or more 14 days after inoculation and does not increase 28 days after inoculation from the level of 14 days after inoculation. The aforementioned criteria are B criteria, which are one of the criteria when the preservative efficacy test described in the EUROPEAN PHARMACOPOEIA 7.0 (EUROPEAN PHARMACOPOEIA 7.0, Efficacy of antimicrobial preservation) is performed (hereinafter to be referred to as "EP-B criteria"), and having the immediate effectiveness means being compatible with the EP-B criteria.

The operation method of the preservative efficacy test described in the EUROPEAN PHARMACOPOEIA 7.0 includes use of bacteria (*Staphylococcus aureus* and *Pseudomonas aeruginosa*) and fungi *(Candida albicans* and *Aspergillus brasiliensis (niger))* as test microorganisms, and the following operations (i) - (iv). Where necessary, microorganisms such as *Escherichia coli* and the like can be added to the test microorganism.
(i) The above-mentioned 5 kinds of microorganism strains to be used for the test are inoculated on the surface of a slant agar medium and precultured. As the agar medium for preculture, a soybean casein digest agar medium is used for bacteria and a Sabouraud glucose agar medium is used for fungi. Bacteria are precultured at 30 - '35°C for 18 - 24 hr, *Candida albicans* is precultured at 20 - 25°C for 40 - 48 hr, and *Aspergillus brasiliensis (niger)* is precultured at 20 - 25°C for 1 week or until good sporulation is achieved.
(ii) An aqueous liquid composition to be subjected to the test is used as a sample, and the sample is dispensed to 5 sterilized stoppered test tubes by 10 mL each. The test microorganism of (i) is inoculated at 10⁵ - 10⁶ cells/mL to prepare a mixed sample and the sample is preserved at 20 - 25°C with protection from light. The test microorganisms are inoculated singly to the sample without mixing.
(iii) After preservation for 24 hr from the start of the preservation, 1 mL of each mixed sample is taken, and the solution is diluted with saline (9 mL). Similar dilution is performed 2 - 3 times and each diluted solution (1 mL) is dispensed to a sterilized petri dish.
(iv) Next, a lecithin 0.1 (w/v)%, polysorbate 80 0.7 (w/v)%-added soybean casein digest agar medium was added to the bacteria, a lecithin 0.1 (w/v)%, polysorbate 80 0.7 (w/v)%-added Sabouraud glucose agar medium was added to the fungi, and the mixtures were cultured under the following conditions. The colony forming units were measured, and a theoretical viable cell count per 1 mL of the mixed sample is calculated.
   culture conditions of bacteria: 30 - 35°C, about 3 days
   culture conditions of fungi: 20 - 25°C, about 5 days

After the operation of the above-mentioned (i) - (iv), when the viable cell counts of all the above-mentioned bacteria (*Staphylococcus aureus, Escherichia coli* and *Pseudomonas aeruginosa*) in the mixed solution decrease by 1 log or more 24 hr after inoculation, decrease by 3 log or more 7 days after inoculation, and do not increase 28 days after inoculation from the level of 7 days after inoculation, and the viable cell counts of fungi *(Candida albicans* and *Aspergillus brasiliensis (niger))* decrease by 1 log or more 14 days after inoculation and do not increase 28 days after inoculation from the level of 14 days after inoculation, the presence of immediate effectiveness of the preservative efficacy is acknowledged.

In the present specification, unless otherwise specified, a composition excellent in stability refers to a composition showing no change in the appearance from the start of both a photostability test and a heat stability test.

A photostability test can be performed by, for example, the following method. A sample (10 mL) is filled in a colorless transparent glass ampoule, and the ampoule is preserved in a photostability test chamber. After preservation, the property of the sample to light is observed under an inspection lamp or fluorescent lighting.

The photostability test chamber is set under the following conditions.
temperature: 25.0°C
illumination: 3.0 klx
overall illumination: 600 klx·h

A heat stability test can be performed by, for example, the following method. A sample (10 mL) is filled in a colorless transparent glass ampoule, and the ampoule is preserved with protection from light in a thermo-hygrostat set at 40°C for 4 months or 6 months. After preservation, the property of the sample to heat is observed under an inspection lamp or fluorescent lighting.

No change in the appearance means that the appearance of the emulsion is white, and has a color equivalent to neutral white (N, 9.5) defined in the JIS Names of Colors (in accordance with JIS Z 8102, Japanese Standards Association, 2nd edition, 2nd printing).

The composition of the present invention is prepared by preparing an aqueous phase containing an emulsifier and an oil phase containing difluprednate, and mixing and emulsifying these phases. For uniform emulsification, a known means such as a homomixer, a homogenizer, a high-pressure homogenizer, an ultra high-pressure homogenizer (microfluidizer) and the like can be used. Other additives such as tonicity agent, buffering agent and the like, including zinc, may be dissolved in an aqueous phase of an emulsifier or added to an emulsion after emulsification.

Particularly, it is desirably produced by a step of preparing an aqueous phase by adding zinc, an emulsifier, a tonicity agent and a buffering agent to water, a step of preparing a difluprednate oil phase by dissolving difluprednate in oil, a step of preparing a coarse emulsion by mixing the aqueous phase and the difluprednate oil phase, and coarsely emulsifying the mixture by a homogenizer and the like, and a step of preparing an oil-in-water emulsion by micronizing the coarse emulsion by a homogenizer. To suppress a decrease in the stability of difluprednate in the production step, the aqueous phase is desirably adjusted to pH 5 - 7 by adding a pH adjuster, before mixing it with the difluprednate oil phase.

In the present specification, the "emulsification" refers to processing an oil phase into a number of ultrafine droplets and dispersing and maintaining them in an aqueous phase. The "coarse emulsification" refers to one form of emulsification, wherein an oil phase is processed into fine droplets of a certain level and dispersed and maintained in an aqueous phase. In this case, the size of the droplet is not uniform. The "micronization" refers to one form of emulsification, wherein a coarse emulsion is further processed using a device such as a microfluidizer and the like to further micronize the droplets of the oil phase to have a size uniform to some extent.

In the preparation step of the composition of the present invention, zinc, and additives such as a tonicity agent, a buffering agent and the like may be dissolved in an aqueous phase or added to an emulsion after emulsification.

The composition of the present invention is preferably used as a preparation for topical administration to the eye, nose, ear or skin, and further as an ophthalmic composition such as an eye drop and the like, a nasal drop, an ear drop or a lotion.

The composition of the present invention has an excellent anti-inflammatory action, an excellent antiallergic action and an excellent antimicrobial action. Accordingly, the composition is useful for the prophylaxis or treatment of various inflammatory diseases or allergic diseases such as allergic conjunctivitis, spring catarrh, marginal blepharitis, catarrhal conjunctivitis, uveitis, inflammation or pain caused by ophthalmic surgery, macular edema and the like. In addition, the composition can be also advantageously used for topical administration to eye, nose, ear, skin and the like.

The composition of the present invention can be safely administered to mammal (human, dog, rabbit, bovine, horse, monkey, cat, sheep etc.).

While the dose of the composition of the present invention varies depending on the kind and symptom of the disease, the age and body weight of the patients and the like, when it is used, for example, as an eye drop for an adult, an eye drop containing 0.01 - 0.2 (w/v)% of difluprednate is desirably instilled by 1 - 2 drops/dose per one eye of a patient about 2 to 4 times per day according to the symptoms.

In addition, the present invention provides a method of stabilizing an emulsion composition comprising difluprednate, the method comprising preparing an emulsion composition coprising difluprednate and zinc. More particularly, it provides a method of stabilizing an emulsion composition comprising difluprednate, the method comprising mixing (a) difluprednate, (b) water, (c) oil, (d) emulsifier and (e) zinc to give an oil-in-water emulsion composition (hereinafter to be referred to as the method of the present invention). In addition, sorbic acid and boric acid may be further added to the emulsion composition.

Difluprednate that can be used for the method of the present invention are as described above.

Examples of the kind of the oil usable for the method of the present invention include castor oil, peanut oil, cottonseed oil, soybean oil, olive oil, medium-chain triglyceride [e.g., Miglyol (trade name, Mitsuba Trading Co., Ltd.)] and the like as mentioned above. More preferred are castor oil, medium-chain triglyceride (e.g., Miglyol) and the like showing high solubility of difluprednate, and particularly preferred is castor oil.

As the kind of emulsifier that can be used for the method of the present invention, the aforementioned non-ionic surfactant and the like can be mentioned. Examples thereof include polyoxyethylene sorbitan ester of fatty acids, polyoxyethylene hydrogenated castor oils, alkyl aryl polyether alcohol type polymers, polyoxyethylene fatty acid esters, polyoxyethylene polyoxypropylene glycols or sucrose fatty acid esters, preferably polyoxyethylene sorbitan monooleate, polyoxyethylene sorbitan monolaurate, polyoxyethylene sorbitan monopalmitate, polyoxyethylene sorbitan monostearate, polyoxyethylene sorbitan tristearate, polyoxyethylene hydrogenated castor oil 10, polyoxyethylene hydrogenated castor oil 40, polyoxyethylene hydrogenated castor oil 50, polyoxyethylene hydrogenated castor oil 60, tyloxapol, polyoxyl stearates and the like. Particularly preferred are polysorbate 80, polyoxyethylene hydrogenated castor oil 60, tyloxapol and polyoxyl 40 stearate. These may be used in combination.

The zinc in the method of the present invention is preferably a compound that can be zinc ion in the emulsion composition. Zinc may be in the form of a zinc salt or a zinc complex.

Examples of the compound that can be zinc ion include metal zinc, zinc salt, zinc complex and the like.

The metal zinc may be nanoparticulate zinc or colloidal zinc.

Examples of the zinc salt include zinc sulfate, zinc chloride, zinc oxide, zinc acetate, zinc carbonate, zinc hydroxide, zinc citrate, zinc lactate, zinc phosphate, zinc gluconate and the like. The zinc salt may be a hydrate thereof.

Examples of the zinc complex include zinc-ethanolamine, zinc-dimethyldithiocarbamate, zinc-sulfate, zinc-2-ethylhexanoate, zinc-quaternary alkylammonium, zinc-nitrate, zinc-8-quinolinolate, zinc amino acid complex and the like.

A preferable example of the zinc salt or zinc complex includes at least one selected from the group consisting of zinc sulfate and zinc chloride.

The content of zinc in the method of the present invention when it is a zinc salt or zinc complex is generally not less than about 0.00001 (w/v)% and not more than about 0.1 (w/v)%, preferably not less than about 0.0005 (w/v) and not more than about 0.1 (w/v), more preferably not less than about 0.001 (w/v)% and not more than about 0.1 (w/v)%, of the composition as the concentration of zinc ion.

The content of sorbic acid in the method of the present invention is not particularly limited, and it is not less than 0.01 (w/v)% and not more than 0.5 (w/v)%, preferably not less than 0.05 (w/v)% and not more than 0.2 (w/v)%, more preferably 0.1 (w/v)%, of the composition.

The content of boric acid in the method of the present invention is not particularly limited, and it is not less than 0.05 (w/v)% and not more than 0.5 (w/v)%, preferably not less than 0.1 (w/v)% and not more than 0.5 (w/v)%, more preferably 0.1 (w/v)%, of the composition.

In the method of the present invention, various additives such as the aforementioned tonicity agent, buffering agent, preservative other than zinc, stabilizer, antioxidant, chelating agent, pH adjuster, thickener and the like may be further used. The detail of such various additives is as described above.

The present invention is explained in more detail in the following by referring to Examples. It is needless to say that the present invention is not limited by the Examples. Examples

### Experimental Example 1 Preservation effectiveness test

### 1) Preparation method

Castor oil (50 g) was weighed and heated in a beaker (water bath temperature: 85 - 95°C). Thereto was added difluprednate (0.5 g) and the mixture was stirred to give solution A containing difluprednate dissolved therein. Separately, polysorbate 80 (40 g) and concentrated glycerol (22 g) were weighed in a beaker, and water (350 mL) was added. This solution was heated to about 65 - 75°C, sodium acetate hydrate (0.5 g) was added with stirring, and the mixture was stirred to give solution B. Solution B heated to about 70°C was stirred (3000 rpm) by a homomixer (T.K. ROBOMIX, PRIMIX Corporation). Solution A heated to about 90°C was added dropwise by small portions to stirring solution B. The whole amount of solution A was added, the rotation number of the homomixer was set to 8000 rpm, and the mixture was stirred at about 70°C for 1 hr. This solution was cooled to room temperature, and a suitable amount of 1 mol/L aqueous sodium hydroxide solution was added to adjust pH to about 5.5. Purified water was added to this solution to a total amount of 500 mL to give a coarse emulsion. Then, the coarse emulsion was micronized using Microfluidizer (M-110EH, Microfluidics Corp.) to give about 400 mL of an emulsion stock solution (pressure about 1500 kgf/cm², temperature 35 - 45°C, number of passes 20 times). The prepared amount was appropriately changed as necessary.

The emulsion stock solution (50 mL) was taken in a 100 mL beaker, and zinc sulfate hydrate was added to make the final concentration of zinc ion 0.00001 (w/v)%. This solution was adjusted to pH about 5.5, and purified water was added to the total amount of 100 mL to give the emulsion of Example 1. The emulsions of Example 2 and Comparative Examples 1 - 4 were prepared in the same manner as in Example 1.

### 2) Test method

As the test microorganism, bacteria (*Staphylococcus aureus* (ATCC 6538), *Escherichia coli* (ATCC 8739) and *Pseudomonas aeruginosa* (ATCC 9027)) were used. In addition, fungi *(Candida albicans* (ATCC 10231) and *Aspergillus brasiliensis (niger)* (ATCC 16404)) were used. Each test microorganism was inoculated on the surface of a slant agar medium and precultured. The three kinds of bacteria were cultured in a soybean casein digest agar medium at 30 - 35°C for 18 - 24 hr. *Candida albicans* was cultured in a Sabouraud glucose agar medium at 20 - 25°C for 40 - 48 hr. *Aspergillus brasiliensis (niger)* was cultured in a Sabouraud glucose agar medium at 20 - 25°C for 1 week or until good sporulation was obtained.

An emulsion to be subjected to the test was used as a sample, and the sample was dispensed to 5 sterilized stoppered test tubes by 10 mL each. The test microorganism was added to the sample such that the cell count was 10⁵ - 10⁶ cells/mL to give a mixed sample. The test microorganism was singly added to the sample. The mixed sample was preserved at 20 - 25°C with protection from light.

At 6 hr, 24 hr, 7 days, 14 days and 28 days from the start of the preservation, 1 mL of each mixed sample was taken, and the solution was diluted with saline (9 mL). Similar dilution was performed 2 - 3 times and each diluted solution (1 mL) was dispensed to a sterilized petri dish. 0.1 (w/v)% lecithin, 0.7 (w/v)% polysorbate 80-added soybean casein digest agar medium was added to the bacteria, and the mixture was cultured at 30 - 35°C for about 3 days. 0.1 (w/v)% lecithin, 0.7 (w/v)% polysorbate 80-added Sabouraud glucose agar medium was added to fungi and the mixture was cultured at 20 - 25°C for about 5 days. After culture, the colony forming units were measured, and the viable cell count per 1 mL of the mixed sample was calculated.

### 3) Criteria

The judgment followed the criteria of the Japanese Pharmacopoeia (JP), 16th Edition, preservative efficacy tests, Category I products. That is, when the viable cell count of the bacteria in the mixed solution 14 days later decreased to 0.1% or less of the inoculated cell number, and 28 days later, was of the level equivalent to or not more than that 14 days later, "preservative efficacy" was acknowledged. When the viable cell count of fungi in the mixed solution 14 days and 28 days later was of the level equivalent to or not more than the inoculated cell number, "preservative efficacy" was acknowledged. When the formulation was found to have preservative efficacy against all the test microorganism strains, it was judged to be "compatible".

### 4) Test results

As shown in Table 1, an emulsion having excellent preservative efficacy (compatible with the Japanese Pharmacopoeia) could be prepared by adding an antimicrobial metal (zinc, silver, copper) to the emulsion. Emulsions added with benzalkonium chloride or chlorhexidine gluconate generally used for aqueous liquids (eye drop) failed to show satisfactory preservative efficacy.

**Table 1**

| component | amount (g) | | | | | |
|---|---|---|---|---|---|---|
| | Example 1 | Example 2 | Comparative Example 1 | Comparative Example 2 | Comparative Example 3 | Comparative Example 4 |
| | emulsion | emulsion | emulsion | emulsion | emulsion | emulsion |
| difluprednate | 0.05 | 0.05 | 0.05 | 0.05 | 0.05 | 0.05 |
| castor oil | 5.0 | 5.0 | 5.0 | 5.0 | 5.0 | 5.0 |
| polysorbate 80 | 4.0 | 4.0 | 4.0 | 4.0 | 4.0 | 4.0 |
| concentrated glycerol | 2.2 | 2.2 | 2.2 | 2.2 | 2.2 | 2.2 |
| sodium acetate hydrate | 0.05 | 0.05 | 0.05 | 0.05 | 0.05 | 0.05 |
| zinc sulfate hydrate | 0.00001 as Zn²⁺ | 0.0005 as Zn²⁺ | | | | |
| silver nitrate | | | 0.005 as Ag⁺ | | | |
| copper sulfate pentahydrate | | | | 0.001 as Cu²⁺ | | |
| benzalkonium chloride | | | | | 0.01 | |
| chlorhexidine gluconate | | | | | | 0.01 |
| purified water | q.s. | q.s. | q.s. | q.s. | q.s. | q.s. |
| sodium hydroxide | q.s. | q.s. | q.s. | q.s. | q.s. | q.s. |
| total amount | 100 mL | 100 mL | 100 mL | 100 mL | 100 mL | 100 mL |
| pH | 5.5 | 5.5 | 5.5 | 5.5 | 5.5 | 5.5 |
| preservative efficacy (JP) | compatible | compatible | compatible | compatible | incompatible | incompatible |

### Experimental Example 2 Stability test

### 1) Preparation method

Each emulsion was prepared by a method similar to that in Experimental Example 1. Each suspension was prepared by the following method. Polysorbate 80 (40 g) and concentrated glycerol (22 g) were weighed in a beaker, and water (400 mL) was added. After dissolution, sodium acetate hydrate (0.5 g) and difluprednate (0.5 g) were added, and the mixture was stirred until difluprednate was uniformly dispersed, and adjusted to pH about 5.5. Purified water was added to the solution to a specified amount to give a suspension stock solution. The suspension stock solution (50 mL) was measured in a beaker, and each metal compound was added to a given final concentration. This solution was adjusted to pH about 5.5, and purified water was added to the total amount of 100 mL to give the object suspension.

### 2) Test method (photostability test)

The prepared sample was filled in a colorless transparent glass ampoule by 10 mL, and preserved in a photostability test chamber. After preservation, the sample was taken out as appropriate, and the apperance of the emulsion and suspension of difluprednate to the light was observed. The observation was performed under an inspection lamp or fluorescent lighting.

The photostability test chamber was set to the following conditions.
temperature: 25.0°C
illumination: 3.0 klx
overall illumination: 600 klx·h

### 3) Test method (heat stability test)

The prepared sample was filled in a colorless transparent glass ampoule by 10 mL, and preserved for 4 months or 6 months in a thermo-hygrostat set to 40°C with protection from light. After preservation, the sample was taken out as appropriate, and the apperance of the emulsion and suspension of difluprednate to the heat was observed. The observation was performed under an inspection lamp or fluorescent lighting.

### 4) Test results

As shown in Table 2, the emulsion containing zinc ion showed no change in the appearance in both the photostability test and the heat stability test as compared to when the test was started, and was stabler than the emulsion containing other metal ion. No change means that the appearance of the emulsion is white, which is a color equivalent to neutral white (N, 9.5) defined in the JIS Names of Colors (in accordance with JIS Z 8102, Japanese Standards Association, 2nd edition, 2nd printing).

In the heat stability test, an emulsion containing silver ion did not show changes in the property and was stable. However, in the photostability test, an emulsion containing silver ion produced black precipitates.

An emulsion containing copper ion turned from pale-yellow white to yellow-white emulsion in both the photostability test and the heat stability test. Different from the results of the emulsion, a suspension containing copper ion did not show changes in the property in the photostability test, and was stable (different stability between suspension and emulsion).

Therefrom it was considered that the photostability and heat stability of an emulsion containing metal ion are not the same as those of a simple aqueous preparation. That is, the stability of an emulsion containing zinc ion is a specific effect found in an emulsion containing difluprednate, unlike other metal ions. As shown in Table 3, moreover, stability of zinc ion could be confirmed even when zinc sulfate hydrate and zinc chloride were used. From the results of Experimental Example 1 and Experimental Example 2, it was found that, when formulating an emulsion containing difluprednate, an emulsion containing zinc ion as a preservative shows both the particularly excellent preservative efficacy and stability.

**Table 2**

| component | amount (g) | | | | | |
|---|---|---|---|---|---|---|
| | Example 3 | Comparative Example 5 | Comparative Example 6 | Comparative Example 7 | Comparative Example 8 | Comparative Example 9 |
| | emulsion | emulsion | emulsion | suspension | suspension | suspension |
| difluprednate | 0.05 | 0.05 | 0.05 | 0.05 | 0.05 | 0.05 |
| castor oil | 5.0 | 5.0 | 5.0 | | | |
| polysorbate 80 | 4.0 | 4.0 | 4.0 | 4.0 | 4.0 | 4.0 |
| concentrated glycerol | 2.2 | 2.2 | 2.2 | 2.2 | 2.2 | 2.2 |
| sodium acetate hydrate | 0.05 | 0.05 | 0.05 | 0.05 | 0.05 | 0.05 |
| Zinc sulfate hydrate | 0.001 as Zn²⁺ | | | 0.001 as Zn²⁺ | | |
| silver nitrate | | 0.001 as Ag⁺ | | | 0.001 as -Ag⁺ | |
| copper sulfate pentahydrate | | | 0.001 as Cu²⁺ | | | 0.001 as Cu²⁺ |
| purified water | q.s. | q.s. | q.s. | q.s. | q.s. | q.s. |
| sodium hydroxide | q.s. | q.s. | q.s. | q.s. | q.s. | q.s. |
| total amount | 100 mL | 100 mL | 100 mL | 100 mL | 100 mL | 100 mL |
| pH | 5.5 | 5.5 | 5.5 | 5.5 | 5.5 | 5.5 |
| initial | white emulsion | white emulsion | white emulsion | white turbid by shaking | white turbid by shaking | white turbid by shaking |
| appearance/photostability test (600 klx·h illumination) | no change | black precipitates | pale-yellow white emulsion | no change | black precipitates | no change |
| appearance/heat stability test (preserved at 40°C for 6 months with protection from light) | no change | no change | yellow-white emulsion | no change | no change | solution turned pale-yellow |

**Table 3**

| component | amount (g) | | |
|---|---|---|---|
| | Example 4 | Example 5 | Example 6 |
| | emulsion | emulsion | emulsion |
| difluprednate | 0.05 | 0.05 | 0.05 |
| castor oil | 5.0 | 5.0 | 5.0 |
| polysorbate 80 | 4.0 | 4.0 | 4.0 |
| concentrated glycerol | 2.2 | 2.2 | 2.2 |
| sodium acetate hydrate | 0.05 | 0.05 | 0.05 |
| zinc sulfate hydrate | 0.1 as Zn²⁺ | | |
| zinc chloride | | 0.001 as Zn²⁺ | 0.1 as Zn²⁺ |
| purified water | q.s. | q.s. | q.s. |
| sodium hydroxide | q.s. | q.s. | q.s. |
| total amount | 100 mL | 100 mL | 100 mL |
| pH | 5.5 | 5.5 | 5.5 |
| initial | white emulsion | white emulsion | white emulsion |
| appearance/photostability test (600 klx·h illumination) | no change | no change | no change |
| appearance/heat stability test (preserved at 40°C for 4 months with protection from light) | no change | no change | no change |

### Experimental Example 3 Preservative efficacy test

### 1) Preparation method

Each emulsion was prepared by a method similar to that in Experimental Example 1.

### 2) Test method

The preservative efficacy test was performed by a method similar to that in Experimental Example 1.

### 3) Criteria

In the same manner as in Experimental Example 1, judgment was made according to the criteria for the Japanese Pharmacopoeia, 16th Edition, preservative efficacy tests, category I products.

### 4) Test results

As shown in Table 4, emulsions containing 0.001 (w/v)% zinc ion (Examples 7 and 9), and an emulsion containing 0.1 (w/v)% zinc ion (Example 8) show excellent preservative efficacy (compatible with the Japanese Pharmacopoeia).

**Table 4**

| component | amount (g) | | |
|---|---|---|---|
| | Example 7 | Example 8 | Example 9 |
| | emulsion | emulsion | emulsion |
| difluprednate | 0.05 | 0.05 | 0.05 |
| castor oil | 5.0 | 5.0 | 5.0 |
| polysorbate 80 | 4.0 | 4.0 | 4.0 |
| concentrated glycerol | 2.2 | 2.2 | 2.2 |
| sodium acetate hydrate | 0.05 | 0.05 | 0.05 |
| sorbic acid | | | 0.1 |
| boric acid | | | 0.1 |
| zinc sulfate hydrate | 0.001 as Zn²⁺ | 0.1 as Zn²⁺ | 0.001 as Zn²⁺ |
| purified water | q.s. | q.s. | q.s. |
| sodium hydroxide | q.s. | q.s. | q.s. |
| total amount | 100 mL | 100 mL | 100 mL |
| pH | 5.5 | 5.5 | 5.5 |
| preservative efficacy (JP) | compatible | compatible | compatible |

### Experimental Example 4 Preservative efficacy test

### 1) Preparation method

The emulsion of Example 10 was prepared by a method similar to that in Experimental Example 1

### 2) Test method

The preservative efficacy test was performed by a method similar to that in Experimental Example 1.

### 3) Criteria

The test followed B criteria, which are one of the criteria for preservative efficacy test in the EUROPEAN PHARMACOPOEIA 7.0 (EUROPEAN PHARMACOPOEIA 7.0, Efficacy of antimicrobial preservation). That is, when the viable cell count of the bacteria in the mixed solution decreased by 1 log or more 24 hr after inoculation, decreased by 3 log or more 7 days after inoculation, and 28 days after inoculation, was of the level equivalent to or not more than that 7 days after inoculation, "preservative efficacy" was acknowledged. When the viable cell count of fungi in the mixed solution decreased by 1 log or more 14 days after inoculation, and 28 days after inoculation, was of the level equivalent to or not more than that 14 days after inoculation, "preservative efficacy" was acknowledged. When the formulation was found to have preservative efficacy against all the test microorganism strains, it was judged to be "EP-B compatible". Simultaneously, judgment by the JP criteria according to the Japanese Pharmacopoeia, 16th Edition (see Experimental Example 1) was also performed.

### 4) Test results

As shown in Table 5, the emulsion containing 0.1 (w/v)% sorbic acid and 0.1 (w/v)% boric acid in addition to 0.1 (w/v)% zinc ion (Example 10) showed improved immediate effectiveness of the preservative efficacy, and the preservative efficacy thereof was compatible with EP-B criteria.

**Table 5**

| component | amount (g) |
|---|---|
| | Example 10 |
| | emulsion |
| difluprednate | 0.05 |
| castor oil | 5.0 |
| polysorbate 80 | 4.0 |
| concentrated glycerol | 2.2 |
| sodium acetate hydrate | 0.05 |
| sorbic acid | 0.1 |
| boric acid | 0.1 |
| zinc sulfate hydrate | 0.1 as Zn²⁺ |
| purified water | q.s. |
| sodium hydroxide | q.s. |
| total amount | 100 mL |
| pH | 5.5 |
| preservative efficacy (EP-B) | compatible |
| preservative efficacy (JP) | compatible |

### Experimental Example 5 preservative efficacy test and stability test

### 1) Preparation method

Each emulsion of Example 11, Example 12, Comparative Example 10 and Comparative Example 11 was prepared by a method similar to that in Experimental Example 1.

### 2) Test method

The preservative efficacy test was performed by a method similar to that in Experimental Example 1, and the photostability test and the heat stability test were performed by the methods similar to those in Experimental Example 2.

### 3) Criteria (preservative efficacy test)

In the same manner as in Experimental Example 1, judgment was made according to the criteria for the Japanese Pharmacopoeia, 16th Edition, preservative efficacy tests, category I products.

### 4) Test results

As shown in Table 6, the emulsions containing 0.001 (w/v)% zinc ion and adjusted to pH 5.5 and pH 7.0 (Examples 11 and 12) showed excellent preservative efficacy (compatible with the Japanese Pharmacopoeia). The emulsion containing 0.2 (w/v)% sorbic acid and adjusted to pH 5.5 (Comparative Example 10) showed excellent preservative efficacy, but the emulsion adjusted to pH 7.0 (Comparative Example 11) did not show satisfactory preservative efficacy. While the preservative efficacy of the emulsion containing sorbic acid is dependent on pH, the emulsion containing zinc ion did not depend on pH and showed excellent preservative efficacy.

The emulsion containing zinc ion showed no change in the appearance as compared to that at the time the test was started in both the photostability test and the heat stability test. No change means, as indicated in Experimental Example 2, that the appearance of the emulsion is white, which is a color equivalent to neutral white (N, 9.5) defined in the JIS Names of Colors (in accordance with JIS Z 8102, Japanese Standards Association, 2nd edition, 2nd printing).

From the results of the preservative efficacy test, photostability test and heat stability test, it was found that an emulsion containing zinc ion as a preservative simultaneously has pH-independent excellent preservative efficacy and excellent stability to both light and heat.

**Table 6**

| component | amount (g) | | | |
|---|---|---|---|---|
| | Example 11 | Example 12 | Comparative Example 10 | Comparative Example 11 |
| | emulsion | emulsion | emulsion | emulsion |
| difluprednate | 0.05 | 0.05 | 0.05 | 0.05 |
| castor oil | 5.0 | 5.0 | 5.0 | 5.0 |
| polysorbate 80 | 4.0 | 4.0 | 4.0 | 4.0 |
| concentrated glycerol | 2.2 | 2.2 | 2.2 | 2.2 |
| sodium acetate hydrate | 0.05 | 0.05 | 0.05 | 0.05 |
| zinc sulfate hydrate | 0.001 as Zn²⁺ | 0.001 as Zn²⁺ | | |
| sorbic acid | | | 0.2 | 0.2 |
| boric acid | | | 0.1 | 0.1 |
| sodium edetate | | | 0.02 | 0.02 |
| purified water | q.s. | q.s. | q.s. | q.s. |
| sodium hydroxide | q.s. | q.s. | q.s. | q.s. |
| total amount | 100 mL | 100 mL | 100 mL | 100 mL |
| pH | 5.5 | 7.0 | 5.5 | 7.0 |
| preservative efficacy (JP) | compatible | compatible | compatible | incompatible |
| initial | white emulsion | white emulsion | white emulsion | white emulsion |
| appearance/photostability test (600 klx·h illumination) | no change | no change | | |
| appearance/heat stability test (preserved at 40°C for 3 months with protection from light) | no change | no change | | |

While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed in the scope of the appended "CLAIMS."

This application is based on a US Provisional Patent Application No. 61/765,307 (filing date: February 15, 2013).

## Claims

1. An emulsion composition comprising difluprednate and zinc.

2. The emulsion composition according to claim 1, wherein zinc is a zinc salt or a zinc complex.

3. The emulsion composition according to claim 2, wherein the zinc salt or zinc complex is at least one selected from the group consisting of zinc sulfate and zinc chloride.

4. The emulsion composition according to claim 2 or 3, wherein the zinc salt or zinc complex has a concentration as zinc ion of not less than 0.00001 (w/v)% and not more than 0.1 (w/v)%.

5. The emulsion composition according to claim 3, further comprising sorbic acid and boric acid.

6. The emulsion composition according to any one of claims 1 to 5, which is an ophthalmic composition.

7. A method of stabilizing an emulsion composition comprising difluprednate, the method comprising preparing an emulsion composition comprising difluprednate and zinc.

8. The method according to claim 7, wherein zinc is a zinc salt or a zinc complex.

9. The method according to claim 8, wherein the zinc salt or zinc complex is at least one selected from the group consisting of zinc sulfate and zinc chloride.

10. The method according to claim 8 or 9, wherein the zinc salt or zinc complex is added to the emulsion composition at a concentration of not less than 0.00001 (w/v)% and not more than 0.1 (w/v)% as zinc ion.

11. The method according to claim 9, further comprising adding sorbic acid and boric acid to the emulsion composition.

12. The method according to any one of claims 7 to 11, wherein the emulsion composition is an ophthalmic composition.

13. The emulsion composition according to any one of claims 1 to 6 for use in the prevention or treatment of an inflammatory disease or allergic disease.

14. The emulsion composition for use according to claim 13 wherein the inflammatory disease or allergic disease is selected from allergic conjunctivitis, spring catarrh, marginal blepharitis, catarrhal conjunctivitis, uveitis, macular edema and inflammation or pain caused by ophthalmic surgery.

15. The emulsion composition according to any one of claims 1 to 6 for use as an antimicrobial agent.

## Patentansprüche

1. Eine Emulsionszusammensetzung, umfassend Difluprednat und Zink.

2. Die Emulsionszusammensetzung gemäß Anspruch 1, wobei Zink ein Zinksalz oder ein Zinkkomplex ist.

3. Die Emulsionszusammensetzung gemäß Anspruch 2, wobei das Zinksalz oder der Zinkkomplex mindestens eines/einer ausgewählt aus der Gruppe bestehend aus Zinksulfat und Zinkchlorid ist.

4. Die Emulsionszusammensetzung gemäß Anspruch 2 oder 3, wobei das Zinksalz oder der Zinkkomplex eine Konzentration, bezogen auf Zinkionen, von nicht weniger als 0,00001 (Gew./Vol.)% und nicht mehr als 0,1 (Gew./Vol.)% aufweist.

5. Die Emulsionszusammensetzung gemäß Anspruch 3, ferner umfassend Sorbinsäure und Borsäure.

6. Die Emulsionszusammensetzung gemäß einem der Ansprüche 1 bis 5, die eine ophthalmologische Zusammensetzung ist.

7. Ein Verfahren zur Stabilisierung einer Emulsionszusammensetzung umfassend Difluprednat, wobei das Verfahren das Herstellen einer Emulsionszusammensetzung, umfassend Difluprednat und Zink, umfasst.

8. Das Verfahren gemäß Anspruch 7, wobei Zink ein Zinksalz oder ein Zinkkomplex ist.

9. Das Verfahren gemäß Anspruch 8, wobei das Zinksalz oder der Zinkkomplex mindestens eines/einer ausgewählt aus der Gruppe bestehend aus Zinksulfat und Zinkchlorid ist.

10. Das Verfahren gemäß Anspruch 8 oder 9, wobei das Zinksalz oder der Zinkkomplex zu der Emulsionszusammensetzung in einer Konzentration von nicht weniger als 0,00001 (Gew./Vol.)% und nicht mehr als 0,1 (Gew./Vol.)%, bezogen auf Zinkionen, gegeben wird.

11. Das Verfahren gemäß Anspruch 9, ferner umfassend Zugeben von Sorbinsäure und Borsäure zu der Emulsionszusammensetzung.

12. Das Verfahren gemäß einem der Ansprüche 7 bis 11, wobei die Emulsionszusammensetzung eine ophthalmologische Zusammensetzung ist.

13. Die Emulsionszusammensetzung gemäß einem der Ansprüche 1 bis 6 zur Verwendung bei der Vorbeugung oder Behandlung einer entzündlichen Erkrankung oder einer allergischen Erkrankung.

14. Die Emulsionszusammensetzung zur Verwendung gemäß Anspruch 13, wobei die entzündliche Erkrankung oder die allergische Erkrankung aus allergischer Konjunktivitis, Frühlingskatarrh, marginaler Blepharitis, katarrhalischer Konjunktivitis, Uveitis, Makulaödem und Entzündung oder Schmerz aufgrund einer ophthalmologischen Operation ausgewählt ist.

15. Die Emulsionszusammensetzung gemäß einem der Ansprüche 1 bis 6 zur Verwendung als ein antimikrobielles Mittel.

## Revendications

1. Composition d'émulsion comprenant du difluprednate et du zinc.

2. Composition d'émulsion selon la revendication 1, dans laquelle le zinc est un sel de zinc ou un complexe de zinc.

3. Composition d'émulsion selon la revendication 2, dans laquelle le sel de zinc ou le complexe de zinc est au moins un sélectionné dans le groupe constitué du sulfate de zinc et du chlorure de zinc.

4. Composition d'émulsion selon la revendication 2 ou 3, dans laquelle le sel de zinc ou le complexe de zinc a une concentration sous la forme d'ion zinc pas inférieure à 0,00001 % (p/v) et pas supérieure à 0,1 % (p/v).

5. Composition d'émulsion selon la revendication 3, comprenant en outre de l'acide sorbique et de l'acide borique.

6. Composition d'émulsion selon l'une quelconque des revendications 1 à 5, qui est une composition ophtalmique.

7. Méthode de stabilisation d'une composition d'émulsion comprenant du difluprednate, la méthode comprenant la préparation d'une composition d'émulsion comprenant du difluprednate et du zinc.

8. Méthode selon la revendication 7, dans laquelle le zinc est un sel de zinc ou un complexe de zinc.

9. Méthode selon la revendication 8, dans laquelle le sel de zinc ou le complexe de zinc est au moins l'un sélectionné dans le groupe constitué du sulfate de zinc et du chlorure de zinc.

10. Méthode selon la revendication 8 ou 9, dans laquelle le sel de zinc ou le complexe de zinc est ajouté à la composition d'émulsion en une concentration pas inférieure à 0,00001 % (p/v) et pas supérieure à 0,1 % (p/v) sous la forme d'ion zinc.

11. Méthode selon la revendication 9, comprenant en outre l'ajout d'acide sorbique et d'acide borique à la composition d'émulsion.

12. Méthode selon l'une quelconque des revendications 7 à 11, dans laquelle la composition d'émulsion est une composition ophtalmique.

13. Composition d'émulsion selon l'une quelconque des revendications 1 à 6 pour son utilisation dans la prévention ou le traitement d'une maladie inflammatoire ou d'une maladie allergique.

14. Composition d'émulsion pour son utilisation selon la revendication 13, dans laquelle la maladie inflammatoire ou la maladie allergique est sélectionnée parmi la conjonctivite allergique, le catarrhe printanier, la blépharite marginale, la conjonctivite catarrhale, l'uvéite, l'oedème maculaire et une inflammation ou une douleur provoquées par la chirurgie ophtalmique.

15. Composition d'émulsion selon l'une quelconque des revendications 1 à 6 pour son utilisation en tant qu'agent antimicrobien.
